# EUROPÄISCHE PATENTANMELDUNG

(11) **EP 2 160 979 A1**
(43) Veröffentlichungstag der Anmeldung: **10.03.2010**
(21) Anmeldenummer: 08015878.5
(22) Anmeldetag: 09.09.2008
(51) Int. Cl.: A61B 5/15

(54) **Individualisierung von medizinischen Hilfsmitteln**

(71) Anmelder: Roche Diagnostics GmbH, 68298 Mannheim (DE); F.HOFFMANN-LA ROCHE AG, 4070 Basel (CH)
(72) Erfinder: Kuhr, Hans-Jürgen, 68219 Mannheim (DE); Konya, Ahmet, 67165 Waldsee (DE)
(74) Vertreter: Silber, Anton

(57) **Zusammenfassung**

Es wird ein Verfahren zur Aufwertung eines medizinischen Hilfsmittels zur Behandlung von Diabetes im home-monitoring Bereich beschrieben, mit den Schritten: Bereitstellen von Verbrauchsmittel in einem Gehäuse mit einem typspezifischen Erkennungs-Code zur Verwendung in einem medizinischen Hilfsmittel, Speicherung des Erkennungs-Codes in Kombination mit einer Benutzer-Identifikation und Ermittlung eines Benutzerstatus, Anbieten einer dem Benutzerstatus entsprechenden Auswahl mindestens einer Ausgestaltung eines Ergänzungsmoduls für das medizinische Hilfsmittel, Generieren eines Auftrags zur Lieferung eines Ergänzungsmoduls aufgrund der Auswahl des Benutzers.

## Beschreibung

### Technisches Gebiet:

Die Erfindung betrifft eine Methode zur Aufwertung von medizinischen Hilfsmitteln.

Für die Benutzer von medizinischen Hilfsmitteln, wie Analysegeräten, Pumpensystemen oder sonstigen diagnostischen und therapeutischen Hilfsmitteln im Home Monitoring Bereich, insbesondere für Diabetes-Patienten, die mehrere Male am Tag Aktionen im Zusammenhang mit ihrer Krankheit ausführen müssen, wird es immer wichtiger, eine leichte und komfortable Handhabung dieser medizinischen Hilfsmittel vornehmen zu können. Ein weiterer zunehmender Trend nicht nur im home-monitoring Bereich ist es, solche Hilfsmittel möglichst für den eigenen Gebrauch und Geschmack individualisiert anpassen zu können.

### Stand der Technik:

Im Stand der Technik wird hierzu in der EP 1 279 365 eine Möglichkeit beschrieben, ein Glukosemeter mit einem zusätzlichen Umgehäuse zu versehen, welches die Funktionen des Glukosemeters nicht beeinträchtigt, aber für den Patienten einen besseren Griff oder unterschiedliche Farbgebung sowie zusätzliche Informationen für das System und damit dem Patienten bereitzustellen. Nachteil dieses zusätzlichen Umgehäuses ist es, dass der Benutzer keine Möglichkeit hat, die Ausgestaltung dieses Umgehäuses zu beeinflussen.

Aus den Nachteilen des Standes der Technik ergibt sich die Aufgabe, dem Benutzer von medizinischen Hilfsmitteln eine Möglichkeit zur Individualisierung seiner krankheitsbezogenen Geräte und Utensilien zu geben.

Diese Aufgabe wird gelöst durch ein Verfahren zur Aufwertung eines medizinischen Hilfsmittels zur Behandlung von Diabetes im Home Monitoring Bereich mit den Schritten:
- Bereitstellen von Verbrauchsmittel in einem Gehäuse mit einem typspezifischen Erkennungscode zur Verwendung in einem medizinischen Hilfsmittel,
- Speicherung des Erkennungscodes in Kombination mit einer Benutzer-Identifikation und Ermittlung eines Benutzerstatus,
- Anbieten einer dem Benutzerstatus entsprechenden Auswahl mindestens einer Ausgestaltung eines Ergänzungsmoduls für das medizinische Hilfsmittel
- Generierung eines Auftrags zur Lieferung eines Ergänzungsmoduls aufgrund der Auswahl des Benutzers.

Auch, wenn dieses Verfahren für das Krankheitsbild Diabetes beschrieben wird, ist eine Anwendung auch für andere home-monitoring Patienten, die beispielsweise ihren Koagulationswert häufig bestimmen müssen oder ihren Urinwert kontrollieren müssen ebenfalls einsetzbar, weshalb die Anmeldung nicht auf Diabetes beschränkt ist.

Mit Hilfe dieses Verfahrens wird dem Benutzer die Möglichkeit gegeben über das Sammeln eines Kredits in Form eines Erkennungs-Codes sich zusätzliche Ergänzungsmodule für medizinische Hilfsmittel selbständig und individuell zusammenstellen zu können. Dabei ist es völlig unabhängig, durch welche Verbrauchsmittel er diesen Kredit, den er beispielsweise durch Addition verschiedener Erkennungs-Codes gewinnen kann, durch Kauf von Verbrauchsmitteln erlangt hat. Das Verfahren bietet dem Benutzer die Möglichkeit den Kredit in Form des Erkennungs-Codes zusammen mit zusätzlichen Informationen in Form der Benutzeridentifikation zu kombinieren und damit eine benutzerspezifische Auswahl mindestens einer Ausgestaltung eines Ergänzungsmoduls für das medizinische Hilfsmittel angeboten zu bekommen. Das System kann aufgrund verschiedener benutzerspezifischer Voraussetzungen beispielsweise aufgrund des Alters oder aber auch des Krankheitsstatus oder sonstiger benutzerspezifischer Daten, ganz individuell unterschiedlichste Zusammenstellungen von Ergänzungsmodulen für den Benutzer zusammenstellen. Da es für verschiedene Benutzergruppen unterschiedliche Anforderungen an die medizinischen Hilfsmittel gibt, kann diesen unterschiedlichen Anforderungen hiermit Rechnung getragen werden.

Dies führt dazu, dass beispielsweise Kindern eine andere farbliche oder formgebende Ausgestaltung der Ergänzungsmodule angeboten werden kann, als dem älteren Benutzer, dem es vor allem auf gute Handhabbarkeit und gute Lesbarkeit von z.B. Messergebnissen ankommt. Neben dem Alter können auch krankheitsspezifische Daten zu einer anders artigen Auswahl führen. So würde einem Diabetes 1 Patienten eine andere Auswahl dargestellt werden als dem Diabetes 2 Patienten. Auch andere Krankheiten können dazu führen, dass zusätzliche Ergänzungsmodule aus diesem Krankheitsumfeld angeboten würden.

Durch das Verfahren ist folglich eine individuelle Ausgestaltung möglich, das bedeutet dass die Anwendergruppe der Kinder beispielsweise durch farbliche Gestaltung dazu animiert werden kann, deutlich häufigere Messungen durchzuführen, da das Gerät mit dem Ergänzungsmodul mehr als Spielzeug ausgestaltet werden kann. Dahingegen könnte ein älterer Benutzer durch ein Ergänzungsmodul, das beispielsweise ein zusätzliches großes Display zur Anzeige gemessener Glukosewerte das Handling seiner Krankheit erleichtert wird. Auf diese Weise kann durch die Individualisierung ein besseres Management des Patienten seiner Erkrankung erreicht werden.

Diese Individualisierung ist aufgrund des vielfältigen Portfolios von bereits bestehenden medizinischen Hilfsmitteln wie Messgeräten, Stechhilfen und Insulinpumpen ohne ein zusätzliches Individualisierungsverfahren nicht möglich, da die Vielfältigkeit an Ausgestaltungsmöglichkeiten sowohl gestalterischer als auch funktionaler Merkmale nicht praktikabel wäre, um sie dem Benutzer direkt beim Kauf eines Gerätes anzubieten. Erst durch die zusätzliche Möglichkeit, dass medizinische Hilfsmittel durch ein Ergänzungsmodul individuell auf die eigenen Bedürfnisse anzupassen, ist es möglich, einer Vielzahl von Benutzergruppen die nötigen und gestalterischen Variationen kostengünstig und praktikabel anbieten zu können.

In einer zweiten Ausführungsform wird ein Individualisierungsverfahren eines handheld Management Systems beschrieben mit den Schritten:
- Bereitstellen einer Auswahl von gestalterischen oder funktionalen Eigenschaften eines Umgehäuses für ein eigenständig funktionsfähiges medizinisches Hilfsmittel, wobei das Umgehäuse das medizinische Hilfsmittel mindestens zum Teil umgibt,
- Registrierung der Auswahl durch den Kunden mindestens einer Eigenschaft des Umgehäuses, **dadurch gekennzeichnet, dass** bei der Auswahl des Umgehäuses die gestalterischen oder funktionalen Eigenschaften des Umgehäuses individuell zusammenstellbar sind, und ein entsprechender Auftrag zur Lieferung des ausgewählten Umgehäuses generiert wird.

Das beanspruchte Verfahren stellt ebenfalls eine Individualisierungsmöglichkeit für den Benutzer dar, wobei in dieser Ausführungsform der Benutzer für ein bereits eigenständig funktionsfähiges medizinisches Hilfsmittel ein Ergänzungsmodul in Form eines Umgehäuses individuell gestalten kann. Dies bietet dem Benutzer die Möglichkeit, zunächst ein medizinisches Hilfsmittel wie beispielsweise eine Stechhilfe oder ein Analysemessgerät zu kaufen, das eigenständig funktionsfähig ist, d. h. alle Funktionen die notwendig sind um beispielsweise eine Blutglucosemessung oder eine Gabe von Insulin durchzuführen, vorhanden sind. Durch das Individualisierungsverfahren hat der Benutzer jedoch nachträglich die Möglichkeit dieses medizinische Hilfsmittel so auszugestalten, dass es für seine individuellen Ansprüche passt. Ein solches eigenständiges funktionsfähiges Modul, dass durch ein zusätzliches Umgehäuse ergänzt werden kann, um weitere Funktionen in das System einzubringen, wird bereits in der EP 1 967 139 in Form einer magazinierten Stechhilfe beschrieben.

Beiden hier beschriebenen Verfahren liegt das Prinzip der Individualisierung von Ergänzungsmodulen, wie Umgehäuse oder zusätzliche Bauteile für das medizinische Hilfsmittel, aber auch zusätzliche Software individuell auswählen und/oder ausgestalten zu können. Dies kann anhand eines Belohnungsverfahrens stattfinden, bei dem der Benutzer durch Kauf von Verbrauchsmitteln einen Kredit erlangen kann, den er über einen Erkennungscode auf dem Verbrauchsmittel sammeln kann und zu gegebener Zeit in Form eines Ergänzungsmoduls für ein medizinisches Hilfsmittel einlösen kann. In beiden Fällen kann der Benutzer durch zusätzliche Bezahlung seinen Kredit variieren.

In diesem Zusammenhang ist es erstrebenswert, eine Vielzahl variabel gestalteter Verbrauchsmitteleinheiten, z.B. in Form eines Magazins verschiedener Verbrauchsmittel wie Stechelemente, Testelemente oder Insulinmengen unabhängig ihrer Menge an Verbrauchsmittel mit dem medizinischen Hilfsmittel funktional wechselwirken zu lassen. Dies kann durch ein System realisiert werden, das ein medizinisches Hilfsmittel, welches mit einem Magazin wechselwirken kann sowie ein Magazin, das Verbrauchsmittel beinhaltet und in das medizinische Hilfsmittel einlegbar ist, enthält. Das System ist **dadurch gekennzeichnet, dass** das Magazin mit einer im ungebrauchten Zustand unterschiedlichen Anzahl an Verbrauchsmitteln versehbar ist.

In der EP 1 967 139 wird diese Bevorratung von Lanzetten in Form eines Bandes in einem Magazin vorgesehen, wobei durch die Ausgestaltung der Magazinierung in Form eines Bandes, das zwischen 2 Rollen bewegbar gelagert ist, die Möglichkeit besteht, die Anzahl der Lanzetten zu variieren, indem beispielsweise die Abstände zwischen den Lanzetten oder insgesamt die Bandlänge in dem Magazin variiert werden kann, ohne dass das Magazin eine andere Form oder Größe aufweisen muss. Die Gehäusegröße kann bei einer Magazinierung zwischen 1 und ca. 400 Lanzetten die gleiche Gehäusegröße aufweisen.

Dieses Prinzip der bandförmigen Magazinierung kann nicht nur für Stechelemente sondern auch für bspw. Testelemente, integrierte Lanzetten mit Testelementen oder andere magazinierbare Verbrauchsmittel verwendet werden. Hierdurch wird erreicht, dass beispielsweise die Ausgestaltung eines Umgehäuses nicht an die selbständig funktionsfähige Stechhilfe mit integriertem Magazin bei unterschiedlicher Menge an Stechelementen in der Größe angepasst werden muss. Hierdurch wird zusätzlich eine kostengünstigere Bereitstellung von sehr unterschiedlich ausgestalteten Umgehäusen möglich gemacht. Folglich bietet sich durch die Ausgestaltung des beschriebenen Systems eine Möglichkeit, ein Individualisierungsverfahren wie zuvor beschrieben umzusetzen.

Auf diese Art und Weise kann eine besondere Belohnung desjenigen Benutzers vorgenommen werden, der aufgrund seiner Krankheit einen hohen Verbrauch an Verbrauchsmittel aufweist. Da das bevorzugte System unabhängig von der Menge an Verbrauchsmitteln immer mit dem gleichen medizinischen Hilfsmittel wechselwirken kann, können verschiedene Befüllungsgrade von Magazinen bereitgestellt werden, ohne dass der Benutzer ein anderes medizinisches Gerät benötigt. Dies kann aufgrund einer einheitlichen Größe des Magazins erreicht werden, unabhängig von der Anzahl oder Menge des Verbrauchsmittels. Alternativ kann eine Adaptionsmodul vorgesehen sein, dass entweder an dem Magazin angebracht ist oder an dem medizinischen Hilfsmittel, um unterschiedlich große Magazine in die gleiche Aufnahmestelle des Hilfsmittels anbringen zu können. Die Funktionsweise von Magazin und medizinischem Hilfsmittel ist dadurch nicht beeinträchtigt. Dabei kann die Kredithöhe, die in dem Erkennungs-Code abgespeichert ist, entweder linear mit der Menge an Verbrauchsmittel steigen oder sich unproportional erhöhen, z.B. schneller mit größerer Menge an bereitgestelltem Verbrauchsmittel pro Magazin.

Das erfindungsgemäße Aufwertungs- oder Individualisierungsverfahren kann mit Hilfe eines Computerprogramms ausgeführt werden, das entweder auf einem Datenträger, wie CD-Rom, USB-Stick oder Diskette zur Verfügung gestellt wird, oder über ein Netzwerk, z. B. über Internet für den Benutzer erreichbar gemacht wird.

Dabei wird dem Benutzer beispielsweise eine Eingabemaske zur Verfügung gestellt, in die er die benötigten Daten, wie Erkennungs-Code und / oder Benutzer-Identifikation eingeben kann, um mit Hilfe dieser Daten eine Auswahl von Produkten angezeigt zu bekommen, die seinem Benutzerstatus, der aus der Information des Erkennungs-Code und / oder der Benutzer-Identifikation ermittelt wurde, entspricht.

Dabei kann das Programm eine Vielzahl von Möglichkeit aufweisen, wie die Auswahl dem Benutzer präsentiert wird. Eine Alternative ist, dass der Benutzer verschiedene Auswahlanzeigen aufgrund eines Erkennungs-Codes mit seiner Benutzer-Identifikation generiert bekommt. Eine andere Alternative ist, dass der Benutzer eine fiktive Auswahl von Benutzerstati generieren kann, die ihn interessiert. Hierdurch erlangt der Benutzer die Gelegenheit, sich Produktkombinationen anschauen zu können, die ihn interessieren, die er sich jedoch mit seinem jetzigen Kredit noch nicht leisten kann. Dadurch kann er motiviert werden seinen Kredit zu erhöhen und damit einen besseren Zustand seiner Krankheitsüberwachung und Therapie zu erreichen. Das Programm bietet weiterhin die Möglichkeit, persönliche Daten und Kreditbeträge abzuspeichern, die erst später in Form einer Lieferung umgesetzt werden.

Alternativ kann sich der Benutzer auch sämtliche gespeicherte Produkte (in Form von Ergänzungsmodulen) anzeigen lassen, um zu entscheiden, ob er weitere Erkennungs-Codes hinzufügen möchte, um eine größere Auswahl zu erhalten.

Dabei kann die Benutzer-Identifikation wahlweise für die Auswahl der angezeigten Produkte einbezogen werden oder vernachlässigt werden.

In einem weiteren Schritt wird dem Benutzer die Möglichkeit gegeben, seine Wahl in einen Auftrag auszugestalten, der die Lieferung des ausgewählten Ergänzungsmoduls zur Folge hat.

Vorteil dieses Verfahrens ist, dass der Benutzer jederzeit die Möglichkeit hat, seine bestehenden Utensilien, die er für das Handling seiner Krankheit benötigt, wie beispielsweise Analysegeräte, (Accu-Chek® Active®, Aviva®, Compact®), Stechhilfen (Accu-Chek ® Multiclix®), Insulinpumpen (Accu-Chek ® Spirit®), Datenverarbeitungsgeräte (Accu-Chek® Smart Pix®), individuell nach seinem Geschmack und für seine Ansprüche erweitern und / oder ausgestalten kann.

Selbst, wenn er zurzeit keinen Kredit in Form eines Erkennungs-Codes vorrätig hat, kann er sich einen Überblick über die Möglichkeiten der Ausgestaltung seiner Geräte / Utensilien und deren Ausgestaltung in Form eines Ergänzungsmoduls verschaffen.

Hierzu besitzt das Eingabemodul eine Möglichkeit, einen fiktiven Erkennungs-Code einzugeben oder sich Produkte für verschiedene Benutzerstati anzeigen zu lassen, ohne einen Erkennungs-Code real erworben zu haben.

### Medizinisches Hilfsmittel

Als medizinisches Hilfsmittel können alle Analysegeräte, Stechhilfen, Datenverarbeitungssysteme, Insulinpumpensysteme, kontinuierliche Messgeräte, sowie jegliche Utensilien, die ein Benutzer im Home Monitoring Bereich zur Behandlung seiner Erkrankung gebraucht, angesehen werden. Diese medizinischen Hilfsmittel werden vorzugsweise mit Verbrauchsmitteln bestückt, wobei das Verbrauchsmittel mit einem Erkennungscode versehen werden kann oder direkt bei Kauf des Verbrauchsmittels zu einer Auswahl durch den Patienten eines Ergänzungsmoduls, z.B. in Form eines Umgehäuses für das medizinische Hilfsmittel führen kann. Das medizinische Hilfsmittel ist bevorzugter Weise bereits ohne das Ergänzungsmodul funktionsfähig und kann durch ein Ergänzungsmodul aufgewertet oder individualisiert werden. Das Ergänzungsmodul kann hierbei ebenfalls ein medizinisches Hilfsmittel sein. Dieses kann ebenfalls selbständig funktionsfähig sein.

### Verbrauchsmittel

In Abhängigkeit des verwendeten medizinischen Hilfsmittels können die Verbrauchsmittel in Form von z.B. Stechelementen, wie Lanzetten, analytischen Testelementen, Kathetern, Insulinkartuschen oder sonstigen Utensilien, die ein Diabetiker zur Behandlung seines Diabetes in Zusammenhang mit einem medizinischen Hilfsmittel benötigt, vorliegen. Die Ausgestaltung dieser Verbrauchsmittel ist dabei nicht limitiert und kann jede Form bzw. Funktionalität, wie vielfältig im Stand der Technik bekannt, annehmen. Es kann beispielsweise auch eine mehrfache Verwendung stattfinden. Bevorzugterweise handelt es sich jedoch um disposible Einheiten.

Die Verbrauchsmittel sind vorzugsweise in einem Gehäuse untergebracht, wie beispielsweise einem Magazin oder einem Vorratsbehältnis. Dies können auch Plastik oder Glaskartuschen zur Bevorratung von Flüssigkeiten sein. Dabei können die Verbrauchsmittel entweder einzeln entnommen und mit dem Hilfsmittel wechselwirken oder bei Verwendung eines Magazins auch direkt in Magazinform mit dem Hilfsmittel wechselwirken, so dass eine Bereitstellung des Verbrauchsmittels durch das Hilfsmittel bei Benutzung des medizinischen Hilfsmittel direkt erfolgen kann. Als Verbrauchsmittel kann weiterhin auch jegliches Zusatzmittel für das medizinische Hilfsmittel angesehen werden, wie z.B. ein zusätzliches Umgehäuse oder sonstige Ergänzungsmodule für das medizinische Hilfsmittel. Bevorzugter Weise wechselwirkt das Umgehäuse so mit dem medizinischen Hilfsmittel, dass es dieses mindestens zu einem Teil umgibt. Hierdurch kann sowohl das Erscheinungsbild, aber auch der Funktionsumfang des medizinischen Hilfsmittels verändert werden.

Da auch medizinische Hilfsmittel wie Stechhilfen, Analysegerät und Insulinpumpen keine unendliche Lebensdauer haben, können sie im Sinne der Erfindung auch als Verbrauchsmittel angesehen werden, folglich mit einem Erkennungscode versehen sein. In diesem speziellen Fall ist das medizinische Hilfsmittel zugleich das Verbrauchsmittel.

### Ergänzungsmodul

Im Sinne der Erfindung ist unter Ergänzungsmodul jegliches zusätzliche Element zu verstehen, das dem Benutzer beim Management seiner Erkrankung dienlich sein kann. Dies können Komponenten sein, die unabhängig von dem medizinischen Hilfsmittel verwendet werden können, oder direkt eine mechanische oder elektronische Verbindung mit dem medizinischen Hilfsmittel aufnehmen können. Beispielsweise können sie über die normale Funktionsweise des medizinischen Hilfsmittels hinaus an dem medizinischen Hilfsmittel angebracht oder darin aktiviert oder implementiert werden kann, oder mit dem medizinischen Hilfsmittel in irgendeiner Form wechselwirken. Unter wechselwirken können alle mechanischen oder elektronischen Wechselwirkungen zwischen dem medizinischen Hilfsmittel und dem Ergänzungsmodul verstanden werden.

Das Ergänzungsmodul kann entweder ein Hardware Modul (z.B. Umgehäuse mit oder ohne weitere Funktionen, eine Tasche, Griffflächen), aber auch ein Software Modul sein. Das Software-Modul kann beispielsweise bereits auf dem medizinischen Hilfsmittel implementiert jedoch beim Kauf des Hilfsmittels noch nicht aktiviert sein und durch die Bereitstellung des Erkennungscodes direkt auf dem medizinischen Hilfsmittel frei geschaltet werden. Alternativ kann durch elektronische Übermittlung des Erkennungs-Codes an eine Zentrale oder Datenbank, die im Zusammenhang mit der Benutzer-Identifikation das Merkmal elektronisch frei geschaltet, das Ergänzungsmodul aktiviert werden. Diese Freischaltung kann durch Übermittlung eines weiteren Codes an den Benutzer erfolgen, der diesen in das medizinische Hilfsmittel eingibt, es kann aber auch direkt elektronisch auf das Hilfsmittel erfolgen, wenn dieses dazu ausgestaltet ist.

Eine weitere alternative Möglichkeit den Transfer von Codes und Aufträgen zu gestalten, ist die Übermittlung via Mobiltelefon oder ähnlichen elektronischen Geräten, die eine Sendefunktion besitzen. Auf diese Weise braucht das medizinische Hilfsmittel lediglich über eine Schnittstelle mit dem Sendegerät verfügen, um den Code oder Auftrag zu übermitteln. Dies kann beispielsweise über eine normale Hardware Schnittstelle wie bspw. USB oder über eine Bluetooth oder Infrarot-Schnittstelle erfolgen.

Das Ergänzungsmodul ist in einer bevorzugten Ausführungsform ein Umgehäuse, welches sowohl gestalterische als auch funktionale Komponenten enthalten kann. Es kann sich jedoch auch um zusätzliche Ergänzungsmodule wie beispielsweise eine Taschenlampe, ein Laserpointer oder ein USB Stick handeln, die modular oder funktional mit dem medizinischen Hilfsmittel interagieren können. Weitere Ergänzungsmodule, die nicht mit dem medizinischen Hilfsmittel direkt wechselwirken, sind eine Erweiterung um einen Stauraum z.B. für Testelemente oder eine Ergänzung um eine Anzeigevorrichtung oder ein Display.

Bevorzugter weise wird das Umgehäuse jedoch mit zusätzlichen Ergänzungsmodulen versehen, die eine zusätzliche funktionale Komponente für das medizinische Hilfsmittel darstellen. Zu dieser Gruppe gehören beispielsweise
1. ein zusätzliches Umgehäuse zur Vergrößerung des Systems zur besseren Handhabung beispielsweise durch ältere Personen,
2. Die Ankoppelung eines elektrischen Antriebs zum automatischen Spannen beispielsweise einer Stechhilfe,
3. Erweiterung um ein Zählwerk für gebrauchte und/oder frische Lanzetten bzw. Testelemente,
4. Erweiterung um einen Sensor zur Prüfung des Fingerandrucks,
5. Erweiterung um ein Blutglukosemessgerät,
6. Elektronischer Auslösemechanismus für eine Stechhilfe,
7. elektronische Anzeige für die Menge unverbrauchter Verbrauchsmittel,
und vieles mehr.

### Erkennungscode

Der Erkennungscode, der sich auf, an oder in den verschiedenen Arten von Verbrauchsmitteln befinden kann, kann jegliche Form von bildlicher oder elektronischer Abbildung von Information sein. Dabei kann der Erkennungscode auch Information über den Typ des Verbrauchsmittels sowie dessen Menge enthalten. Beispiele hierfür sind optische oder elektronische Barcodes, Kennziffern, aufgeklebte Punktezahlen oder sonstige Mittel, die von dem Benutzer bzw. elektronischen Hilfsmitteln in optischer oder elektronischer Form auslesbar sind. Bei der elektronischen Auslesung kann dies durch ein zusätzliches Gerät gewährleistet sein, oder durch das Gerät, in das die Verbrauchsmittel eingebracht werden.

Auch die Weiterverarbeitung z.B. in Form von Speicherung des Erkennungscodes kann entweder rein mechanisch durch den Benutzer erfolgen, indem er die aufgeklebten Bilder, bspw. in Form von Punkten, auf ein Formular überträgt, mit dem er anschließend eine Bestellung vornimmt. Es kann aber alternativ auch durch eine Ankoppelung des medizinischen Hilfsmittels, welches die Erkennungscodes abgespeichert hat, eine direkte Übertragung auf einen Computer oder ein Netzwerk stattfinden, in dem die Daten beispielsweise über Bluetooth oder sonstige elektronische Übermittlungsmethoden auf das Endverarbeitungsgerät übermittelt werden. Auf dem Endverarbeitungsgerät, das entweder das medizinische Hilfsmittel selber oder ein Computer, aber auch eine Datenbank bzw. ein Eingabeprogramm sein kann, wird der Erkennungs-Code mindestens zeitweise gespeichert und weiterverarbeitet. Diese Weiterverarbeitung kann wie bereits erwähnt auch die Schritte des Kombinierens einer Benutzer-Identifikation (wenn vorhanden) und/oder die Bereitstellung einer Auswahl von Produkten beinhalten.

Der Erkennungs-Code enthält Kreditinformationen, die es dem Benutzer ermöglichen mit Hilfe dieses Codes Ergänzungsmodule für medizinische Hilfsmittel zu bestellen. Die Information verschiedener Erkennungs-Codes können miteinander kombiniert werden, und können so für den Benutzer zu einer anderen bzw. erweiterten Auswahl bei den Ergänzungsmodulen führen.

### Benutzer-Identifikation/Benutzerstatus

Der Erkennungscode kann zusammen mit einer Benutzer-Identifikation dazu benutzt werden, eine Auswahl mindestens einer Ausgestaltung eines Ergänzungsmoduls für das medizinische Hilfsmittel bereitzustellen. Hierbei kann die Benutzerinformation lediglich eine Benutzererkennung z.B. in Form des Namens oder einer Erkennungsnummer oder sonstige Information sein.

Im Falle einer Freischaltung eines bereits vorhandenen Merkmals auf dem medizinischen Hilfsmittels, kann diese Benutzer-Identifikation eine Information (bspw. Kennung oder Typenname eines Gerätes) zu dem medizinischen Hilfsmittel sein.

Weiterhin können persönliche Daten als Benutzer-Identifikation hinzu gefügt werden, die bspw. das Alter des Benutzers, bestimmte Vorlieben des Benutzers, Kenntnis über bisher verwendete Geräte bzw. Utensilien durch den Benutzer oder andere zur Person gehörende Daten sein.

Dabei kann diese Benutzer-Identifikation zusätzlich Informationen über den Krankheitszustand des Benutzers enthalten, so dass diese Information zusammen mit dem Erkennungscode dazu verwendet wird, in dem Auswahlprogramm eine differenziertere Auswahl für den Benutzer bereitzustellen. Diese Information über den Krankheitszustand kann beispielsweise auf dem Gebiet der Diabetes die Information darüber sein, ob der Benutzer ein Patient des Typ 1 oder Typ2 Diabetes ist, ob er insulin- oder nicht insulinabhängig ist, welche Systeme er bisher verwendet hat und welche weiteren Krankheiten er hat.

Liegt das Auswahlprogramm beispielsweise in Form eines Kataloges vor, so können solche krankheitsbezogenen Informationen nur bedingt abgebildet werden, jedoch ist auch hier möglich zwischen potentiell sinnvollen Ergänzungsmodulen für verschiedene Krankheitsgruppen wie beispielsweise Typ 1 Diabetes und Typ 2 Diabetes zu unterscheiden, da der Typ 1 Diabetiker meist insulinabhängig ist, während der Typ 2 Diabetespatient dies noch nicht sein muss. So würde die Auswahl für den Typ 2 Diabetespatienten alle Utensilien in Form von Ergänzungsmodulen aus dem Insulinpumpenbereich nicht angeboten bekommen, es sei denn auf seinen speziellen Wunsch hin.

### Gestalterische oder funktionale Komponenten

Zur Individualisierung bestehender Utensilien / Geräte durch den Benutzer kann der Benutzer bei der Wahl eines Ergänzungsmoduls sowohl gestalterische als auch funktionale Komponenten auswählen.

Zu den gestalterischen Komponenten zählen vor allem:
1. Farbe
2. Muster
3. optische Effekte
4. Größe des Ergänzungsmoduls zur optischen Aufwertung
5. Haptik (z.B. Oberflächenbeschaffenheit)
6. Ergonomie (Formgebung)

Diese gestalterischen Komponenten dienen vor allem dazu, um eine Individualisierung für das Ergänzungsmodul und damit auch für die Geräte vorzunehmen. Hiermit soll eine bessere Identifikation des Benutzers mit seinen Utensilien im Zusammenhang mit seiner Krankheit bekommen. Dies soll das Bewusstsein des Benutzers was das richtige Verhalten im Umgang mit seiner Krankheit angeht, erhöhen. Dazu gehört eine regelmäßige Bestimmung des Glucosespiegels im Blut für den Diabetiker, oder die bessere therapeutische Einstellung durch Insulin.

Die funktionalen Komponenten können sein:
1 Weitere Geräte, wie Stechhilfen, Analysegerät, Insulinpumpe, die mit dem bestehenden System bzw. Gerät funktional gekoppelt werden können.
2. Zusätzliche Verbrauchsmittel, wie Stechelemente, Testelemente etc.
3. Variation der Größe des Ergänzungsmoduls zur angepassten Handhabung des medizinischen Hilfsmittels (z. B. bei Sehbehinderten oder älteren Menschen).
4. Zusätzliches Umgehäuse, um weitere Funktionen in das medizinische Hilfsmittel einzubringen, wie
   - zusätzlicher Auslöseknopf für ein Messgerät oder Stechhilfe
   - automatische Anzeige unbenutzter Verbrauchsmittel
   - Display zur Anzeige diverser Ergebnisse und sonstiger Daten
und vieles mehr.

### Beschreibung der Figuren

Figur 1:
   Schematische Zeichnung eines Aufwertungsprozesses eines medizinischen Hilfsmittels mit Hilfe eines Erkennungscodes.
Figur 2:
   Schematische Darstellung eines Individualisierungsprozesses für ein Umgehäuse
Figur 3:
   Darstellung eines medizinischen Home Monitoring Systems mit unterschiedlicher Anzahl von Verbrauchsmitteln.

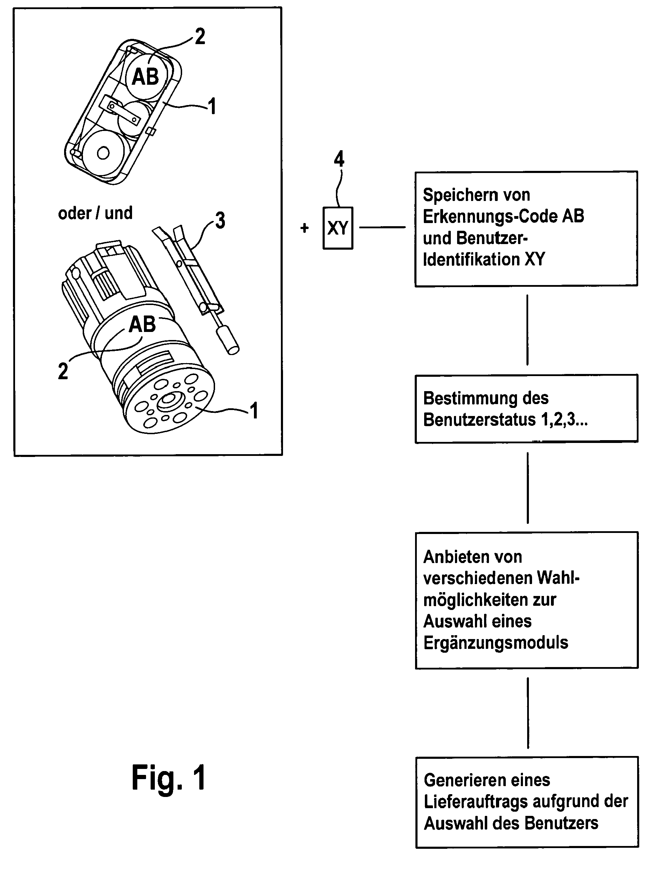

In Figur 1 ist schematisch der Ablauf der Verfahrensschritte zur Aufwertung eines medizinischen Hilfsmittels dargestellt. Hierbei wird zunächst ein Verbrauchsmittel (1), in diesem Beispiel als Magazin für Testelemente oder für Lanzetten (3) dargestellt, bereitgestellt. Auf diesem Verbrauchsmittel (1) ist ein Erkennungscode (2) angebracht, der zusammen mit der Benutzeridentifikation (4) auf einem Datenverarbeitungsgerät (z.B. einem Computer, einer Datenbank oder einem Internetserver) gespeichert wird, oder durch den Benutzer auf einem Formular notiert wird. Auf diesem Datenverarbeitungsgerät oder Formular wird mithilfe des Erkennungscodes (2) und der Benutzeridentifikation (4) ein Benutzerstatus aus einer Reihe von abgespeicherten bzw. abgedruckten Benutzerstati (1, 2, 3 .... X) elektronisch bzw. händisch bestimmt. Aufgrund dieses Benutzerstatus werden dem Benutzer verschiedene Wahlmöglichkeiten zur Auswahl eines Ergänzungsmoduls angeboten. Dies kann in Form einer Auflistung von Produktnamen zusammen mit dem nötigen Kredit zur Erlangung dieses Ergänzungsmoduls geschehen, oder alternativ kann auch eine bildliche Darstellung der verschiedenen Ergänzungsmodule stattfinden, was insbesondere für die gestalterische Ausgestaltung des Ergänzungsmoduls sinnvoll ist. Hierbei kann der Benutzer eine Kombination von verschiedenen Komponenten des Ergänzungsmoduls wählen, z.B. die Farbe mit einem bestimmten Muster sowie weiteren Funktionen für ein Ergänzungsmodul kombinieren.

Bei einer elektronischen Verarbeitung der Daten kann dem Benutzer zu jeder Zeit der nötige Kreditbetrag für seine Kombination angezeigt werden und mit dem eingegebenen Erkennungscode bzw. Benutzerstatus verglichen werden.

Hat der Benutzer eine Auswahl getroffen, kann er im nächsten Schritt aufgrund seiner Auswahl einen Lieferauftrag generieren. Dies kann bei Verwendung eines elektronischen Auswahlmoduls in Form eines elektrischen Auftrags geschehen oder bei einem Katalog als Auswahlmodul in Form eines Lieferauftrags durch ein Formular, auf dem die nötigen Daten wie Erkennungscode und Benutzeridentifikation eingetragen wurden.

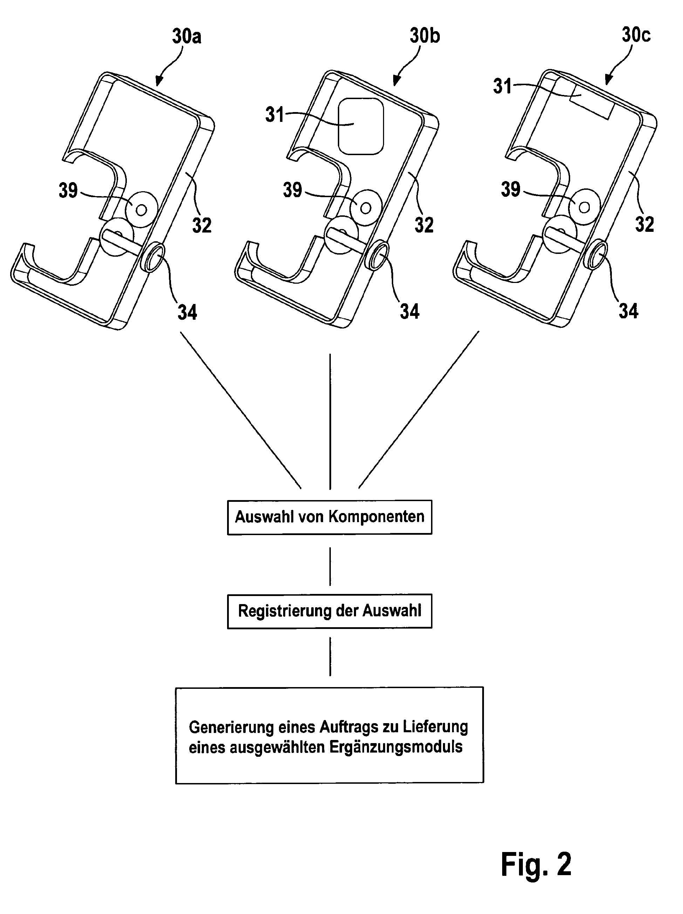

In der Figur 2 werden 3 verschiedene Ausführungsformen eines Umgehäuses (30a, 30b, 30c) gezeigt, die sich aufgrund ihrer Funktionalität bzw. Ausgestaltung voneinander unterscheiden. Ausgehend von dem Umgehäuse (30a) ohne ein Zusatzelement (31), kann das Umgehäuse diverse Zusatzelemente (31) besitzen, wie beispielsweise für das Umgehäuse (30b) in Form eines Displays (31) gezeigt ist oder alternativ dazu das Umgehäuse (30c) ein Zusatzelement (31) in Form einer USB Schnittstelle besitzt. Dies soll beispielhaft die Bereitstellung einer Auswahl von gestalterischen oder funktionalen Eigenschaften eines Umgehäuses für ein eigenständig funktionsfähiges medizinisches Hilfsmittel darstellen wie dies in diesem Fall eine Stechhilfe wäre (die hier nicht gezeigt ist). Dieses Umgehäuse (30a, 30b, 30c) kann beispielsweise weiterhin eine Gehäusewand (32), einen Auslöser (34), eine Schnittstelle (36) sowie einen Antrieb (39) beinhalten.

Nach der Auswahl von Komponenten durch den Benutzer wird diese Auswahl registriert, dies kann durch Aufzeichnung der Auswahl durch den Benutzer in einem Formular geschehen oder bevorzugter weise durch das Auswahlprogramm auf einem Computer oder in einer Datenbank. Aufgrund dieser Registrierung kann dann ein Auftrag zur Lieferung des ausgewählten Ergänzungsmoduls generiert werden, wenn der Benutzer dies möchte. Bevorzugter weise wird auch hier wie bei dem System unter Figur 1 beschrieben, eine Benutzeridentifikation z.B. in Form einer Adresse oder einer Benutzerkennung benötigt, um dem Benutzer sein ausgewähltes Ergänzungsmodell zukommen zu lassen.

Diese in Figur 2 beispielhaft dargestellte Auswahlmöglichkeiten für ein Umgehäuse sind wie unter Figur 1 bereits beschrieben sowohl auf elektronischem wie auch schriftlichem Wege möglich. Die Möglichkeiten der Darstellung von verschiedenen gestalterischen wie auch funktionalen Komponenten kann bei einem elektronischen Datenverarbeitungsprogramm bzw. einer Datenbank deutlich einfacher und bequemer für den Benutzer dargestellt werden.

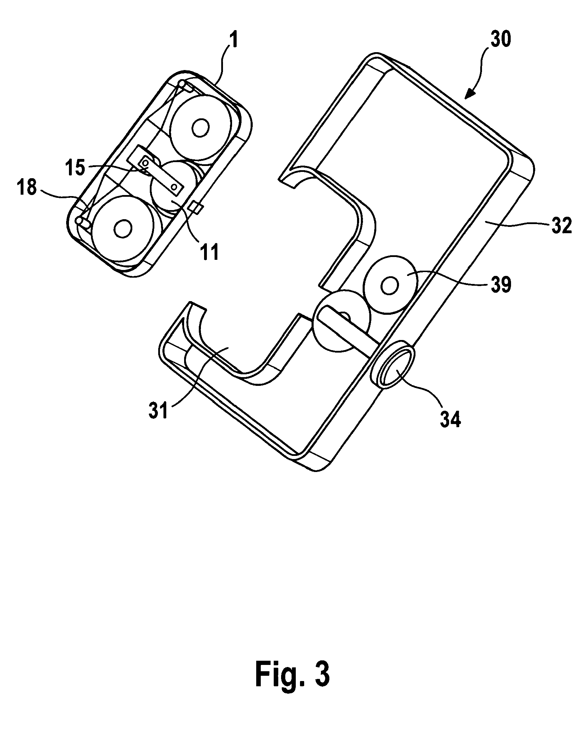

Die Figur 3 zeigt ein Verbrauchsmittel (1) in Form eines Magazins für beispielsweise Lanzetten oder Testelemente. Die Lanzetten oder Testelemente sind auf einem Band (18) befestigt, das durch einen Antriebsmechanismus (15) zur Einstichposition bewegt wird und zum Weitertakten ein Antriebsmechanismus (11) besitzt, um das Band bis zum nächsten Verbrauchsmittel weiter zu bewegen. Durch einen speziellen Vorschubmechanismus kann unabhängig von der Anzahl der Verbrauchsmittel (1) wie beispielsweise Testelemente oder Lanzetten, die gleiche Magazinierungsform und Größe vorgenommen werden. Dies hat den Vorteil, dass das Umgehäuse (3) wie in Figur 3 dargestellt, nicht zusätzlich auf die Größe der Stechhilfe bzw. Testelementmagazinierung angepasst sein muss, sondern eine universelle Größe zumindest für die Aussparung (31) vorgesehen sein kann. Dies führt zu einer Rationalisierung der Produktionskosten, die dem Benutzer dadurch zugute kommen kann, dass er zusätzliche Merkmale für ein Ergänzungsmodul (30) auswählen kann, ohne große Kosten auf sich nehmen zu müssen.

## Patentansprüche

1. Verfahren zur Aufwertung eines medizinischen Hilfsmittels zur Behandlung von Diabetes im home-monitoring Bereich mit den Schritten:
- Bereitstellen von Verbrauchsmittel in einem Gehäuse mit einem typspezifischen Erkennungs-Code zur Verwendung in einem medizinischen Hilfsmittel,
- Speicherung des Erkennungs-Codes in Kombination mit einer Benutzer-Identifikation und Ermittlung eines Benutzerstatus,
- Anbieten einer dem Benutzerstatus entsprechenden Auswahl mindestens einer Ausgestaltung eines Ergänzungsmoduls für das medizinische Hilfsmittel,
- Generieren eines Auftrags zur Lieferung eines Ergänzungsmoduls aufgrund der Auswahl des Benutzers

2. Verfahren gemäß Anspruch 1, **dadurch gekennzeichnet, dass** der Erkennungs-Code Informationen über die Menge der Verbrauchsmittel beinhaltet.

3. Verfahren gemäß einem der vorherigen Ansprüche, **dadurch gekennzeichnet, dass** das medizinische Hilfsmittel eine Stechhilfe, ein Analysegerät oder ein Insulin-Injektionsmodul ist.

4. Verfahren gemäß einem der vorherigen Ansprüche, **dadurch gekennzeichnet, dass** die Ausgestaltung des Ergänzungsmoduls gestalterische und/oder funktionale Komponenten enthält.

5. Verfahren gemäß einem der vorherigen Ansprüche, **dadurch gekennzeichnet, dass** das Ergänzungsmodul ein Umgehäuse ist und das medizinische Hilfsmittel abnehmbar mindestens zu einem Teil umgibt.

6. Verfahren gemäß einem der vorherigen Ansprüche, **dadurch gekennzeichnet, dass** die Benutzer-Identifikation Information über den Krankheitszustand des Benutzers enthält.

7. Verfahren gemäß einem der vorherigen Ansprüche, **dadurch gekennzeichnet, dass** die Auswahl des Ergänzungsmoduls in Abhängigkeit des Benutzerstatus angeboten wird.

8. Verfahren gemäß Anspruch 4 **dadurch gekennzeichnet, dass** die funktionalen Komponenten zu der Gruppe
- zusätzlicher Auslöseknopf
- automatische Anzeige unbenutzter Lanzetten
- Zusätzliche Verbrauchsmittel, wie Stechelemente, Testelemente etc.
- Variation der Größe des Ergänzungsmoduls zur angepassten Handhabung des medizinischen Hilfsmittels.
- automatische Anzeige unbenutzter Verbrauchsmittel
- Display zur Anzeige diverser Ergebnisse und sonstiger Daten
gehören.

9. Verfahren zur Individualisierung eines hand-held Diabetes Management Systems mit den Schritten:
- Bereitstellen einer Auswahl von gestalterischen oder funktionalen Eigenschaften eines Umgehäuses für ein eigenständig funktionsfähiges medizinisches Hilfsmittel, wobei das Umgehäuse das medizinische Hilfsmittel mindestens zum Teil umgibt,
- Registrierung der Auswahl durch den Kunden mindestens einer Eigenschaft des Umgehäuses,
**dadurch gekennzeichnet, dass** bei der Auswahl des Umgehäuses die gestalterischen oder funktionalen Eigenschaften des Umgehäuses individuell zusammenstellbar sind und ein entsprechender Auftrag zur Lieferung des ausgewählten Umgehäuses generiert wird.

10. Verfahren gemäß Anspruch 9, **dadurch gekennzeichnet, dass** das Umgehäuse bei Verwendung mit dem medizinischen Hilfsmittel mit dem Hilfsmittel funktional wechselwirkt.

11. Medizinisches home-monitoring System für die Analyse oder Therapie eines Diabetikers enthaltend:
- ein medizinisches Hilfsmittel, das mit einem Magazin wechselwirken kann,
- ein Magazin, das Verbrauchsmittel beinhaltet und in das medizinische Hilfsmittel einlegbar ist,
**dadurch gekennzeichnet, dass** das Magazin mit einer im ungebrauchten Zustand unterschiedlichen Menge an Verbrauchsmitteln versehbar ist.

12. System zur Verwendung in einem Verfahren gemäß Anspruch 1 oder 9 **dadurch gekennzeichnet, dass** in Abhängigkeit von der Menge der Verbrauchsmittel in dem medizinischen Hilfsmittel das Ergänzungsmodul individuell aus unterschiedlichen Merkmalskombinationen auswählbar ist.

13. System gemäß Anspruch 11 , **dadurch gekennzeichnet, dass** das Magazin bei unterschiedlicher Anzahl der Verbrauchsmittel die gleich Gehäusegröße aufweist.

14. Computer oder Netzwerk mit einem Programm zur Auswahl von Ausgestaltungen von Ergänzungsmodulen für medizinische Hilfsmittel, zur Durchführung der Verfahren gemäß den Ansprüchen 1 oder 9.

15. Datenbank zur Bereitstellung von Daten zur Auswahl von Ausgestaltungen von Ergänzungsmodulen für medizinische Hilfsmittel gemäß den Ansprüchen 1 oder 9.
